Europäisches Patentamt

(19) European Patent Office

Office européen des brevets

(11) EP 0 818 744 A2

(12) EUROPEAN PATENT APPLICATION

(43) Date of publication:
14.01.1998 Bulletin 1998/03

(51) Int Cl.6: G06F 17/50, C07K 1/00, C07K 5/08, C12N 9/74

(21) Application number: 97304412.6

(22) Date of filing: 24.06.1997

(84) Designated Contracting States:
AT BE CH DE DK ES FI FR GB GR IE IT LI LU MC NL PT SE
Designated Extension States:
AL LT LV RO SI

(30) Priority: 08.07.1996 GB 9614302
07.08.1996 GB 9616562

(71) Applicant: Proteus Molecular Design Limited
Macclesfield, Cheshire SK11 0JL (GB)

(72) Inventors:
• Young, Stephen Clinton
  Stockport, Cheshire SK4 4DL (GB)
• Murray, Christopher
  Macclesfield, Cheshire SK10 2TT (GB)

(74) Representative: Cockbain, Julian, Dr.
Frank B. Dehn & Co.,
European Patent Attorneys,
179 Queen Victoria Street
London EC4V 4EL (GB)

(54) Process for selecting candidate drug compounds

(57) The invention relates to a process for drug candidate identification, said process comprising the steps of:

(1) obtaining a computerised representation of the three-dimensional structure of a binding site on the surface of a biological macromolecule;

(2) generating a computerised model of the functional structure of said binding site which may be used to identify favourable and unfavourable interactions between the binding site and a drug candidate molecule;

(3) identifying a molecular fragment (or "template" T) capable of placement within said binding site and capable of carrying at least one (preferably a plurality (ie. at least two) and especially preferably at least 3) substituent group, said molecular fragment either being capable of being synthesized from reagent compounds accessible in substituted form whereby to import said substituent groups on synthesis of said molecular fragment or being present in an accessible reagent compound capable of substitution with said substituent groups by reaction with further accessible reagent compounds;

(4) generating a set of lists of accessible reagent compounds (eg. $a_1$-A, $a_2$-A, $a_3$-A, etc, $b_1$-B, $b_2$-B, $b_3$-B, etc, $c_1$-C, $c_2$-C, $c_3$-C, etc), the lists being such that a combination of compounds taken from each list (eg. $a_1$-A, $b_3$-B and $c_{11}$-C) may be reacted to produce a candidate compound comprising said molecular fragment carrying a plurality of substituent groups (eg. $a_1 b_3 c_{11}$T) thereby generating a first virtual library of candidate compounds being the theoretical set of compounds producible by reaction of the members of said lists (ie. $a_1 b_1 c_1$T, $a_1 b_1 c_2$T, $a_1 b_2 c_1$ T etc), each member of each list comprising a component (eg. A,B,C, etc.) common to the other members of that list and a component (eg. $a_1$, $b_1$, $c_1$, etc) unique within that list;

(5) for each said list limiting the number of members thereof using a first set of exclusion rules thereby to generate a restricted second virtual library of candidate compounds, the operation of said first set of rules involving for each member of each list computerised comparison for favourable or unfavourable interactions between said computerised model and a structure comprising said molecular fragment and a substituent deriving from the unique component within said list of that member, the molecular fragment and the computerized model being held in fixed spatial relationship to each other for said comparison;

(6) evaluating and ranking by computer the members of said second virtual library for favourable and unfavourable interactions with said computerised model and thereby generating a restricted third virtual library of candidate compounds ranked as having favourable interactions;

(7) optionally, selecting from said third virtual library at least one further molecular fragment and repeating steps (4), (5) and (6) to generate an alternative third virtual library;

(8) screening said third virtual library using a second set of exclusion rules thereby to generate a restricted fourth virtual library of candidate compounds comprising compounds which are candidates for synthesis and experimental evaluation for drug efficacy;

(9) synthesizing some or all candidate compounds of said fourth virtual library to produce a candidate compound library;

(10) experimentally evaluating the compounds of said candidate compound library for drug efficacy;

(11) analysing the experimental efficacy data generated in step (10) for structure-activity relationship information;

(12) using the information derived in step (11) selecting a revised set of lists of accessible reagent compounds, said lists being expanded to include selected reagents not present in the restricted lists generated in step (5) and optionally restricted to exclude selected reagents present in the restricted lists generated in step (5);

(13) repeating steps (6) and (7) to identify further compounds which are candidates for synthesis and experimental evaluation for drug efficacy;

(14) synthesising and experimentally evaluating said further compounds for drug efficacy;

(15) if required repeating steps (11) to (14) one or more times;

(16) identifying as a lead candidate a compound synthesized and experimentally evaluated as above.

The process of the invention is characterised by the rapid generation of a relatively small set of readily synthesisable candidate compounds with a high success rate in terms of drug efficacy and hence a high predictive value for directing subsequent iterations.

**Description**

FIELD OF THE INVENTION

This invention relates to a process for selecting lead candidate drug compounds, and in particular to such a process in which synthesis of candidate compounds is simplified and minimized and success rate with synthesized compounds is maximized.

BACKGROUND OF THE INVENTION

Drug discovery has been a time and resource consuming exercise. Traditionally, key steps in drug discovery have included the identification of a compound or set of compounds having the desired drug property, the identification of the active structure within such compounds and the identification of a lead candidate, a compound which incorporates that structure and combines adequate activity with acceptable toxicity and synthetic accessibility. By acceptable synthetic accessibility it is meant that the lead compound should be produceable via a synthetic route which is sufficiently straightforward and inexpensive that commercial production of the compound is a viable option.

The identification of active compounds has involved screening of extensive compound libraries for the desired drug property. Recently, the technique known as combinatorial chemistry has offered a moderate cost route to the synthesis of very large compound libraries which can be screened in this manner. Although it is now increasingly being applied to the synthesis of libraries of non-peptide organic molecules, the combinatorial chemistry technique is especially applicable to the production of libraries of peptide and peptoid compounds, and synthesis and testing of such compound libraries can even be automated and operated under computer control. Thus for example an alternative approach to drug discovery using computer-controlled combinatorial chemistry is described by 3-Dimensional Pharmaceuticals Inc. in WO-A-96/08781.

Unfortunately, however, the peptide and peptoid compounds for which such a combinatorial chemistry approach is particularly suited, due to the ease with which peptide molecules can be produced with a multiplicity of sequences on automated peptide synthesizers, often display undesirable pharmacokinetics, such as poor bioavailability.

An alternative approach to drug discovery has also developed over recent years. This approach referred to variously as Structure-Based Drug Design (SBDD) or Computer Aided Molecular Design (CAMD) involves structural analysis of the receptor site for the drug molecule and can involve computerized generation of a molecular structure which is capable of binding to that site, ie. a structure which has an appropriate structural framework to fit within the receptor site and which is so functionalized as to have favourable interactions with selected functional components of the receptor site.

One example of the SBDD system is the PRO_LIGAND system of Proteus Molecular Design Limited. This is described for example by Clark et al in a series of papers J. Comput.-Aided Mol. Design 9: 13-32 (1995), 9: 139-148 (1995), 9: 213-225 (1995) and 9: 381-395 (1995), J. Med. Chem 37: 3994-4002 (1994), and J. Chem. Inf. Comput. Sci. 35: 914-923 (1995).

While highly effective, SBDD serves to generate and assess molecular structures on the basis of predicted activity without particular regard to synthetic accessibility. These molecules must then be made and tested and subsequent optimization to produce a lead candidate may require time consuming, complicated or expensive chemical syntheses.

It has now been recognised that by combining certain of the features of combinatorial chemistry with certain features of SBDD one can produce a drug discovery system in which only a relatively limited compound library need be generated before a range of active compounds is identified, that that range of active compounds may provide sufficient structure-activity relationship information for a lead candidate to be identified with relatively little iteration (ie. relatively little extension of the library that is initially generated and tested), and that the library may be generated on rational principles ensuring that the vast majority of compounds in the library may be synthetically readily accessible.

In other words, in using the process of the invention to generate the structure activity information necessary to identify a lead candidate one may avoid the need to make and test the large compound libraries required by prior art routine screening or by combinational chemistry and, unlike prior art SBDD techniques, the active compounds identified will implicitly be synthetically readily accessible.

Thus viewed from one aspect the invention provides a process for drug candidate identification, said process comprising the steps of:

(1) obtaining a computerised representation of the three-dimensional structure of a binding site on the surface of a biological macromolecule;

(2) generating a computerised model of the functional structure of said binding site which may be used to identify favourable and unfavourable interactions between the binding site and a drug candidate molecule;

(3) identifying a molecular fragment (or "template" T) capable of placement within said binding site and capable of carrying at least one (preferably a plurality (ie. at least two) and especially preferably at least 3) substituent group, said molecular fragment either being capable of being synthesized from reagent compounds accessible in substituted form whereby to import said substituent groups on synthesis of said molecular fragment or being present in an accessible reagent compound capable of substitution with said substituent groups by reaction with further accessible reagent compounds;

(4) generating a set of lists of accessible reagent compounds (eg. $a_1$-A, $a_2$-A, $a_3$-A, etc, $b_1$-B, $b_2$-B, $b_3$-B, etc, $c_1$-C, $c_2$-C, $c_3$-C, etc), the lists being such that a combination of compounds taken from each list (eg. $a_1$-A, $b_3$-B and $c_{11}$-C) may be reacted to produce a candidate compound comprising said molecular fragment carrying a plurality of substituent groups (eg. $a_1 b_3 c_{11} T$) thereby generating a first virtual library of candidate compounds being the theoretical set of compounds producible by reaction of the members of said lists (ie. $a_1 b_1 c_1 T$, $a_1 b_1 c_2 T$, $a_1 b_2 c_1 T$ etc), each member of each list comprising a component (eg. A,B,C, etc.) common to the other members of that list and a component (eg. $a_1$, $b_1$, $c_1$, etc) unique within that list;

(5) for each said list limiting the number of members thereof using a first set of exclusion rules thereby to generate a restricted second virtual library of candidate compounds, the operation of said first set of rules involving for each member of each list computerised comparison for favourable or unfavourable interactions between said computerised model and a structure comprising said molecular fragment and a substituent deriving from the unique component within said list of that member, the molecular fragment and the computerized model being held in fixed spatial relationship to each other for said comparison;

(6) evaluating and ranking by computer the members of said second virtual library for favourable and unfavourable interactions with said computerised model and thereby generating a restricted third virtual library of candidate compounds ranked as having favourable interactions;

(7) optionally, selecting from said third virtual library at least one further molecular fragment and repeating steps (4), (5) and (6) to generate an alternative third virtual library;

(8) screening said third virtual library using a second set of exclusion rules thereby to generate a restricted fourth virtual library of candidate compounds comprising compounds which are candidates for synthesis and experimental evaluation for drug efficacy;

(9) synthesizing some or all candidate compounds of said fourth virtual library to produce a candidate compound library;

(10) experimentally evaluating the compounds of said candidate compound library for drug efficacy;

(11) analysing the experimental efficacy data generated in step (10) for structure-activity relationship information;

(12) using the information derived in step (11) selecting a revised set of lists of accessible reagent compounds, said lists being expanded to include selected reagents not present in the restricted lists generated in step (5) and optionally restricted to exclude selected reagents present in the restricted lists generated in step (5);

(13) repeating steps (6) and (7) to identify further compounds which are candidates for synthesis and experimental evaluation for drug efficacy;

(14) synthesising and experimentally evaluating said further compounds for drug efficacy;

(15) if required repeating steps (11) to (14) one or more times;

(16) identifying as a lead candidate a compound synthesized and experimentally evaluated as above.

Viewed from an alternative aspect the invention provides a method of manufacturing a drug substance, said method comprising the steps of:

(1) obtaining a computerised representation of the three-dimensional structure of a binding site on the surface of a biological macromolecule;

(2) generating a computerised model of the functional structure of said binding site which may be used to identify favourable and unfavourable interactions between the binding site and a drug candidate molecule;

(3) identifying a molecular fragment capable of placement within said binding site and capable of carrying at least one substituent group, said molecular fragment either being capable of being synthesized from reagent compounds accessible in substituted form whereby to import said substituent groups on synthesis of said molecular fragment or being present in an accessible reagent compound capable of substitution with said substituent groups by reaction with further accessible reagent compounds;

(4) generating a set of lists of accessible reagent compounds, the lists being such that a combination of compounds taken from each list may be reacted to produce a candidate compound comprising said molecular fragment carrying a plurality of substituent groups thereby generating a first virtual library of candidate compounds being the theoretical set of compounds producible by reaction of the members of said lists, each member of each list comprising a component common to the other members of that list and a component unique within that list;

(5) for each said list limiting the number of members thereof using a first set of exclusion rules thereby to generate a restricted second virtual library of candidate compounds, the operation of said first set of rules involving for each member of each list computerised comparison for favourable or unfavourable interactions between said computerised model and a structure comprising said molecular fragment and a substituent deriving from the unique component within said list of that member, the molecular fragment and the computerised model being held in fixed spatial relationship to each other for said comparison;

(6) evaluating and ranking by computer the members of said second virtual library for favourable and unfavourable interactions with said computerised model and thereby generating a restricted third virtual library of candidate compounds ranked as having favourable interactions;

(7) optionally, selecting from said third virtual library at least one further molecular fragment and repeating steps (4), (5) and (6) to generate an alternative third virtual library;

(8) screening said third virtual library using a second set of exclusion rules thereby to generate a restricted fourth virtual library of candidate compounds comprising compounds which are candidates for synthesis and experimental evaluation for drug efficacy;

(9) synthesizing some or all candidate compounds of said fourth virtual library to produce a candidate compound library;

(10) experimentally evaluating the compounds of said candidate compound library for drug efficacy;

(11) analysing the experimental efficacy data generated in step (10) for structure-activity relationship information;

(12) using the information derived in step (11) selecting a revised set of lists of accessible reagent compounds, said lists being expanded to include selected reagents not present in the restricted lists generated in step (5) and optionally restricted to exclude selected reagents present in the restricted lists generated in step (5);

(13) repeating steps (6) and (7) to identify further compounds which are candidates for synthesis and experimental evaluation for drug efficacy;

(14) synthesising and experimentally evaluating said further compounds for drug efficacy;

(15) if required repeating steps (11) to (14) one or more times;

(16) identifying as a lead candidate a compound synthesized and experimentally evaluated as above;

(17) manufacturing the compound identified in step (16) above; and, optionally,

(18) admixing the compound manufactured in step (17) above with at least one pharmaceutically acceptable carrier or excipient.

By "accessible" it is meant that the reagent compounds are commercially available or are synthesizable, preferably via relatively simple routes, from commercially available materials, eg. using known synthetic procedures.

By a "virtual library" is meant the set of compounds theoretically attainable by the inter-reaction of the reagents in the reagent lists for that library. By contrast by "compound library" or "candidate compound library" is meant a library of compounds that have been synthesized.

In the process of the invention, the reagent lists in the first iteration can conveniently be limited to select only one reagent of any group in which the unique substituents (ie. $a_1$, $a_2$, $a_3$ etc) are closely analogous (eg. optical isomers, alkylene chain length homologs, equivalently substituted compounds (eg. compounds substituted by different halo atoms), etc). Similarly, while the reagent lists may initially include both commercially available compounds and transformation products of such compounds, for the first iteration (eg. for the first performance of step (6) for a given template) the lists may be limited to exclude reagents which although commercially available have a long delivery time or are particularly expensive as well as transformations which although synthetically feasible are complex, have poor yields, or require significant purification, etc. In the second and further iterations however such analogs or less readily available compounds can be reinstated within the reagent lists where the structure-activity information derived in step (11) suggests that they may also lead to effective compounds.

In this way the process of the invention may be carried out in such a way that expensive reagents or complex synthetic chemistry is only required at a stage where candidate compound efficacy is already established and the drug discovery process is closing in on a lead candidate.

Nonetheless, by including within the reagent lists not just compounds which are readily available commercially but also chemical transformation products of available reagents, the theoretical size of the virtual libraries is vastly expanded and the capability of the process to assess (and if necessary readily synthesize and test) modifications of the compounds revealed to be active is greatly increased.

Computational requirements may also readily be reduced by limiting the precision of the computer evaluation and ranking of the virtual libraries for the initial performance of steps (5) and/or (6), eg. by limiting the conformational freedom of the compound under evaluation or by specifying the position and orientation of the compound within the binding site model. In this way one may only bring in more complex and accurate evaluation systems for subsequent iterations or for sets of library member which would require the use of expensive or less readily accessible reagents for their production. Indeed, this combination of SBDD concepts to screen a virtual library of accessible compounds before synthesis and experimental evaluation of the resulting limited real library may even render the use of highly computationally-demanding programs unnecessary - ie. a "quick and dirty" computational evaluation may be entirely adequate.

If desired, using optional step (7) in the process of the invention one may refine the selection of the template. Thus for example, step (3) of the method of the invention may be effected by selecting as the template an "anchor" group (Anch) suitable for binding to a section of the binding site, eg. an aryl or other lipophilic group capable of binding to a lipophilic region of the binding site, and performing steps (4) to (6) to identify a further set of templates ($T_n$) which can be coupled at a substitution site to the anchor group thereby to generate a virtual library of anchor-template (Anch-$T_n$) compounds, and to evaluate and rank by computer the members of the anchor-template library for favourable and unfavourable interactions with the computerised model (the Binding Site Model) whereby to produce a restricted set of templates ranked as having favourable interactions, one, more or all of which may serve as the molecular fragment for the reiteration of steps (4) to (6). In this way, by a rational selection of the template, the success rate for the subsequent synthesis and testing of the candidate compound library, and hence the predictive value of the structure-activity information derived therefrom which is the hallmark of the process of the present invention, is still further enhanced.

Indeed the process of the invention is characterised by the rapid generation of a relatively small set of readily synthesisable candidate compounds with a high success rate in terms of drug efficacy and hence a high predictive value for directing subsequent iterations. Unlike standard combinational chemistry the technique is not "blind" relying on random success in the testing of a large library and unlike SBDD the technique inherently produces compounds which are readily synthesizable and readily modified to home in on a lead candidate.

While the rational selection of a template starting from a list of anchor-template candidates as discussed above is a preferred aspect of the invention, template selection may be on the basis of knowledge of the macromolecular target or of knowledge of compounds known to bind to or otherwise influence the target. By way of example a template may be designed to mimic the structure and conformation of a compound known to have the desired drug efficacy while itself having a structure that is accessible either by combination of two or more reagents having the "unique/common" component structure referred to above or directly as a readily substitutable reagent compound (a template reagent).

Once the template or templates have been selected, the "common" components of the reagents are implicitly identified either as functional groups that will react with groups on a template reagent or as groups which will react together to generate the template. The reagent lists may then be generated by searching a computer database of available chemicals (such as the ACD database of MDL Informations Systems Inc.). These lists may then be supplemented by inclusion of accessible transformations of the compounds on the lists, eg. oxidation, reduction or substitution products of such compounds as well as salts, esters, and other feasible chemical transformations. If desired, the members of the resulting lists may be grouped into groups deemed likely to have similar properties in any resulting candidate compounds and also ranked in terms of accessibility (ie. expense, delivery delay, complexity of any required transformation, etc).

The limiting of the lists of reagent compounds in step (5) may conveniently, as discussed above, comprise restrictions of groups of compounds deemed analogous, elimination of low accessibility compounds, elimination of compounds with too high a molecular weight, too large a total atom count or with substituent groups thought to possess undesired properties (eg. charge or reactivity with other groups such that template production may be hindered).

The computational comparison used to further limit the lists may be effected on a list by list basis with for each list the selected members of the other lists remaining constant or preferably with the other substituent positions on the template being vacant. Where the comparison does not involve such vacant sites these "selected members" may be chosen on the basis of perceived compatibility with the binding site but conveniently once the first list (preferably the shortest) has been evaluated, a highly compatible member of that list will be the invariant selected member for evaluation of the next list and so on. Advantageously, once highly compatible members of the other lists have been identified, the first list will be reevaluated with highly compatible members of the other lists being the invariant selected members.

Alternatively and much more preferably, the computational comparison may be significantly simplified by preselection of one (or more if necessary) invariant locations and conformations for the template within the binding site model, followed by comparison, on a list by list basis for individual members of the lists and for an incompletely substituted template, eg. the template carrying only the substituent(s) deriving from the individual list member being investigated. In this way, alternative orientations of a list member which satisfies basic requirements such as appropriate size and functionality (eg. charge, lypophilicity, hydrogen bond donor/acceptor, etc.), may be scrutinised to improve the predictive value of the ranking which is the result of the comparison.

For step (1) of the process of the invention, one can conveniently input 3-D structural information about the binding site (eg. X-ray crystallographic analyses) from published sources, preferably sources which are computer-accessible.

The binding site model generated in step (2) conveniently consists of a representation of those regions of the binding site that can be considered capable of molecular interaction with a xenobiotic or other molecule, labelled according to the nature and geometry of the possible interactions, eg. hydrogen bond donor sites, hydrogen bond acceptor sites, aliphatic and aromatic lipophilic sites, ionic and metal-binding sites, etc.

## DETAILED DESCRIPTION OF THE INVENTION

Briefly put, the process of the invention involves the following steps:

- Construction of a virtual combinatorial library based around a template chemistry considered appropriate for the target molecule and amenable to combinatorial synthesis

- Screening of members of the library based on their interaction with a target receptor

- Synthesis and testing of representative elements of the library as single compounds using a variety of synthetic protocols.

With a known target molecule structure, this process can be done efficiently and accurately and overcomes various difficulties associated with applying combinatorial chemistry or Structure-Based Drug Design.

Firstly, the process offers all the advantages of an array based combinatorial library (single compounds, wider variety of chemistries) whilst sidestepping the problem of small library arrays. This is because a very large virtual library is considered and screened computationally, leaving only a small number of compounds to be synthesised and tested.

Secondly, the need to have one synthetic protocol to cover a wide variety of chemistries is relaxed. The synthesis route can be tailored to accommodate a larger range of chemistries than could be considered by an automated method. Solution and solid phase methods can be used with protection and deprotection steps as required. This means that a larger virtual library can be considered and thus the chance of locating active compounds is increased. The process also allows for simple functional group transformations within the starting materials to increase further the diversity of the virtual library.

Thirdly, by restricting the design process to molecules which are accessible by specified synthetic routes, one avoids the problems often associated with rational drug design, ie. uncertain synthetic feasibility and slow feedback between design and experiment. The process yields a set of compounds based around a common template which can be rapidly synthesised and assayed for activity against a given target. Such a set of compounds might form an immediate QSAR (Quantitative Structure Activity Relationship) training set, in contrast to other drug discovery paradigms where further work would be necessary to derive an equivalent QSAR set. A primary advantage over a traditional

7

medicinal chemistry approach, which would involve obtaining a lead by a screening process and then synthesising a large number of analogues to provide an SAR set, is thus one of cost-effectiveness.

In the process of the invention, each member of the virtual library consists of a common template with different substituents attached to it. The substituents are derived from accessible chemical reagents and it is variation in these substituents at each template attachment point which causes the combinatorial explosion in the number of individual molecules in the library. The library has a synthesis route or strategy (sometimes referred to herein as the template chemistry) associated with it whereby individual members are synthesised from available chemical reagents. The template itself can be an available chemical or can be formed during the chemical reactions (e.g. in ring forming reactions). The available reagents may also undergo general molecular transformations before they are attached to the template and become substituents.

The technological aspects of the process may be separated into four stages. The design specification stage determines the constraints which are to be applied and explored during the computational screening of the virtual library. Obviously, these constraints include the actual specification of the library, the 3D structure of the receptor and any specific constraints derived from the receptor to judge the quality of library members. The second stage involves selecting chemical reagents and screening the corresponding substituents which are used to form members of the virtual library. The substituent screening is based on the structure of the receptor. The accepted substituents are further assessed and filtered using a variety of computer-aided techniques and chemical considerations. The third stage involves enumeration of the virtual library, i.e. production of the full library after all rejected substituents have been deleted. The final computational stage of the procedure is to perform simple checks and calculations on the enumerated library and arrive at a ranking of the molecules in the virtual library for synthesis and testing.

Each of these four stages will now be outlined in detail.

The three aspects of design specification which are discussed below are template selection, template positioning, and the design criteria which will be discussed separately despite being inter-related.

Specification of the design criteria involves careful study of the target macromolecule. Thus, decisions need to be taken at this stage about which X-ray structure(s) of the receptor are to be used (if more than one is available), and whether some refinement by molecular dynamics/molecular mechanics needs to be carried out in order to generate a more accurate starting point for molecular design. Typically more than one snapshot of the receptor structure will be used in successive experiments. Also it is necessary at this stage to decide on the key functionalities in the active site with which the substituents on the candidate compounds are to interact. A 'design model' is then generated for each template attachment point, eg. using the Design Model Generation functionality of PRO_LIGAND (see Clark et al. (supra)). A design model consists of a number of interaction sites which originate from specified receptor atoms and may be either vectors (denoting favourable positions and directions for hydrogen bond interactions with the active site) or points (denoting positions of favourable lipophilic contact with the active site) (see Bohm, J. Comput.-Aided Mol. Design 6: 61 and 593 (1992) and Klebe, J. Mol. Biol. 237: 212 (1994)). The vectors and points are labelled to indicate the particular chemistry they represent; thus D-X and A-Y vectors represent potential hydrogen bond donor and acceptor positions respectively. Similarly, L and R sites represent aliphatic and aromatic lipophilic sites respectively. The density, positions and orientations of the interaction sites are encoded in a rule-base which can be edited by the user and is based on a statistical examination of experimentally preferred intermolecular contacts (see Klebe (supra)).

The purpose of the molecular template is to hold in position the substituents which will make hydrogen bonds, lipophilic contacts or other favourable interactions with the binding site. An advantage of using structural information in the choice of the template chemistry is that knowledge of the receptor can be used to increase the chances of the library containing active molecules. A number of important issues can be identified in the selection of the template chemistry.

- The synthetic chemistry associated with the template should be relatively accessible and capable of delivering a wide diversity of substituents at a number of attachment points.

- Ideally, the template itself should be capable of making a number of favourable contacts with the receptor. This aids in establishing the position of the template and increases the likelihood that the library will contain active molecules.

- In some cases it is possible to infer likely templates from known inhibitors or substrates. For example, in the search for a thrombin inhibitor a known inhibitor of thrombin is PPACK (D-phenylalanyl-prolylarginyl-chloromethylketone) which contains a central proline moiety which could be used as a template, or one could choose a known substructure with strong binding (e.g. guanidinium in the S1 pocket of thrombin) which can be pre-positioned and used to search for potential templates (ie. using the "anchor" technique referred to above).

- Templates can be designed de novo, using structure-based techniques. This could mean using a de novo design method or a receptor-based database screening strategy. It is also advantageous to search reaction databases for suitable ring forming reactions which, for example, would give rise to beta-sheet mimetics.

-   It is desirable that the template has restricted conformational freedom so that only limited numbers of alternative positions for the template need be considered.

The process of template selection may thus involve close collaboration between modellers and synthetic chemists, the former providing expertise about the requirements of the templates in terms of molecular interactions at the binding site and the latter giving guidance concerning the synthetic feasibility of any choices made. The result of the template selection process is a set of scaffolds chosen to achieve the best architecture in the active site and to minimise the synthetic effort required to prepare them. In practice, the decision about which templates to pursue will be a balance between the variety of factors discussed above.

It should be emphasized that unlike many of the combinatorial chemistry techniques described hitherto, it is not necessary that the template or the candidate compounds be peptides or peptoids.

Having chosen the set of templates to be used in the active site of interest, the next task is to position the templates appropriately within the site. In principle, there will be a very large number of orientations of a given template in the site (although this number can be reduced if the chosen template makes a specific interaction with the binding site itself). What is required is to select a subset of these positions which place the template in such a way as to facilitate the molecular interactions that will be formed by the substituents once they are attached.

This placement process could be achieved automatically by means of various objective docking protocols based on molecular mechanics or empirically based energy calculations (see Blaney, Perspect. Drug Disc. Des. $\underline{1}$:301 (1993)) or geometric positioning upon interaction sites (see Bohm, J. Comput.-Aided Mol. Des. $\underline{8}$:623 (1994)). The result of template positioning is a position, or number of positions, in 3D coordinate space for each of the templates. The chosen orientations are saved for future reference.

The process of substituent selection involves and/or other chemical compound databases a number of steps

-   Searching the Available Chemicals Directory (ACD) of MDL Information Systems Inc., San Leandro, California, US (and/or other chemical compound databases) to find potential substituents for a given template

-   Computationally screening these potential substituents, eg. using techniques such as those used in the de novo design program, PRO_LIGAND (see Clark et al. (supra))

-   Assessing and deciding on the preferred substituents at each position.

Each of these steps is explained more fully below. It is important to realize that substituents attached to different attachment points are preferably tested independently of each other at this stage. This makes the process of performing detailed 3D checks on a large virtual library computationally efficient. This and other approximations inherent in this approach are discussed below.

Given a positioned template, it is possible to infer for each template attachment point the nature of the interaction (s) the corresponding substituent is to make with the active site (eg. hydrogen bond, lipophilic contact, etc.), the nature of the functional group required for a coupling reaction to the template (eg. acid chloride with a primary amine) and a distance range between the point of attachment to the template and the point of interaction with the active site.

These two (or more) substructural criteria with the associated distance range(s) constitute a viable query for a 3D database search using database searching tools, such as ISIS/3D from MDL Information Systems Inc., Unity from Tripos Associates Inc., and Chem-3D from Chemical Design Ltd., etc. The query can be made more sophisticated through a consideration of potential molecular transformations, or through the imposition of synthetic constraints on allowed chemistries in specified substructures. By using the ACD, the chance that all chosen substituents will be commercially available is maximised. In general, the search carried out should explore the conformational flexibility of the database molecules to ensure that as many as possible of the potential substituents at each position will be retrieved.

For each template attachment point, a file of potential substituents may be saved as 2D structures to a file, eg. in MDL's SD format (see Dalby, J. Chem. Inf Comput. Sci. $\underline{32}$: 244 (1992)) and then the Converter program (available from MSI, San Deigo, California, US) may be used to add the necessary hydrogen atoms and generate 3D coordinates for the structures.

The methods used for the computational screening of potential substituents may conveniently be techniques such as those used in the de novo design package PRO_LIGAND (see Clark et al (supra)). As described earlier, each template attachment site has its own design model and the template attachment sites themselves are appended to the design models, according to the labels specified in the template file which is input to the program. By automatically labelling the potential substituents for each template attachment position with appropriate interaction link sites, it is possible to use rapid algorithms to establish whether they can form good molecular interactions with the active site. For more details, see Clark, J. Comput.-Aided Mol. Des. 9:13 (1995) and Murray, J. Comput.-Aided Mol. Des. 9:381 (1995).

The flexibility of this approach is enhanced by the ability to detect specified functional groups and replace them with another group. This increases the diversity in the virtual library that is computationally screened and so increases the chance of finding active compounds. The computational deprotection of protected functional groups is one example of how this feature might be used.

The molecular transformation may be controlled by rules containing a SMILES-like notation (see Weininger, J. J. Chem. Inf. Comput. Sci. 28:31 (1988)) for the substructures together with a number of integers. Thus, for instance, one rule may indicate that up to three silyl ethers are to be replaced by hydroxyl groups in any molecule. The geometry for the transformed part of the molecule is rebuilt atom by atom using a rule-based procedure and then relaxed with a molecular mechanics minimisation.

A similar approach is used to protonate or deprotonate certain functional groups specified by the user in order that the molecules to be placed in the active site have realistic protonation patterns. Once the molecules have been subjected to all the transformations requested by the user, they are passed on to the initial molecular property screens.

Before subjecting the potential substituents to more computationally demanding subgraph isomorphism and directed tweak checks, some rapid molecular property screens may be used to eliminate unsuitable structures. Thus, acceptable ranges may be set for a number of properties, eg.

- Molecular weight

- Number of atoms

- Log P (eg. calculated using the method of Viswanadhan, J. Chem. Inf. Comput. Sci. 29:163 (1989))

- Number of rotatable bonds

Any substituents which fall outside the acceptable ranges may thus be automatically rejected. This is useful, for example, when the database entry contains more than one component. The program should automatically separate the components and treat each one as separate substituent. The screen based on the number of atoms tends to remove the undesirable component which is often a counterion. The code can also screen out duplicates.

A further initial screen on substituents may be employed for some complex template chemisties. Thus for example, if in a ring forming reaction one chemical reagent gives rise to two substituents on the template, then the two corresponding template attachment points will have the same list of available chemicals associated with them. Specific checks should ensure that only chemical reagents which have provided a substituent to pass all screens for the first template attachment point are considered for the provision of substituents for the second template attachment site.

The first step in subgraph isomorphism matching process is to label the potential substituent with the appropriate interaction sites. This is accomplished by means of a rule-based procedure where each rule denotes a substructure in the SMILES-like notation mentioned earlier and indicates if and how each of the atoms in that substructure should be labelled. Thus for example, one rule might instruct the program to search the substituent for any matches to a particular specified substructure (eg. C(=NH)N(H)H) and to label the second and fourth atoms of the match as X sites and the third and fifth atoms of the match as D sites. A powerful regular expression-based syntax is available within the SMILES-like notation which permits very flexible definitions of the rules; for instance, a further rule might indicate that any OH or NH group attached to a carbon atom should be labelled as a donor group.

In addition to the interaction sites described earlier, it is also desirable to label each substituent with link sites. These denote the vector site in the structure where the potential substituent will join to the template. The link sites are assigned in an identical manner to the interaction sites. Thus, for instance, a further rule might instruct the program to label the C-C bond in a $CCO_2H$ substructure as a link site (link site vectors are denoted V-W). (Note however that a link site does not have to correspond to an attachment point in an actual chemical reaction; for example, the formation of a peptide bond may be the chemical reaction associated with substituent attachment, but by defining the template to already contain the peptide bond, the C-C bond can be used as the computational link site. The chosen definition is dictated by convenience or computational efficiency, although in templates derived from ring forming reactions, it is often essential to choose link sites which do not correspond to the bond formed in the chemical reaction.)

If, for any reason, a potential substituent cannot be assigned either interaction sites or link sites, it is automatically rejected. Otherwise, the program should proceed to seek a 3D match between the interaction/link sites of the substituent and the interaction/link sites of the design model. This may be accomplished using the subgraph isomorphism algorithm of Ullmann (see J. ACM 23:31 (1976)) which has been used successfully in many chemical structure applications. In order to account for the conformational flexibility of the substituents in this process, distance bounds matrices are calculated using the directed tweak routines which seek to establish the maximum and minimum distances that can be attained between all pairs of atoms through rotation about rotatable bonds (see Murray, J. Comput.-Aided Mol. Des. 9:381 (1995)). The subgraph isomorphism algorithm then uses these distance ranges in establishing a match in the manner described by Clark, J. Mol Graphics 10:194 (1992).

If no match is found for the substituent, it is rejected and the algorithm returns to consider the next available substituent.

The finding of a match for a substituent in the subgraph isomorphism check is not necessarily a sufficient condition for a substituent to be accepted. This is because the distance bounds matrix does not include correlation effects, i.e. the effect that one interatomic distance having one value might have on the possible values attainable by the other interatomic distances. Thus, in order to establish whether the substituent is in fact a viable one for the template attachment point in question, a specific matching conformation should be generated using some form of conformational exploration procedure (see Clark, J. Mol. Graphics 10:194 (1992)).

The procedure adopted for the experimental trials reported below is based on the directed tweak algorithm (see Hurst, J. Chem. Inf. Comput. Sci. 34:190 (1994)) which was originally developed for 3D database searching applications, where it has been shown to be both efficient and effective. Its utility in the field of de novo design has recently been demonstrated (see Murray, J. Comput.-Aided Mol. Des. 9:381 (1995)).

The directed tweak algorithm takes the match established by the subgraph isomorphism algorithm and then seeks to verify it by performing a torsional optimisation of the rotatable bonds in the substituent. After a potential match has been located, the substituent is attached to the template. The bond where attachment occurs is treated as rotatable. The following cost function is minimised by a steepest descent method:

$$F = \frac{1}{\sqrt{N}} \sum_i a_i d_i{}^2$$

where the summation occurs over all N interaction sites, and $d_i$ is the distance between the ith substituent interaction site and the design model interaction site with which it is matched. $a_i$ is a coefficient which depends upon the type of interaction site being matched and is a simple function of the tolerances used in the subgraph isomorphism algorithm. This cost function differs from that used by Hurst (J. Chem. Inf Comput. Sci. 34:190 (1994)) and Murray (supra), in that the distances between pairs of sites are not included, only the absolute distance between the two matched sites. This is possible because the template attachment site provides a fixed point of reference in the design model coordinate space. This means that there are fewer terms in the cost function expression and it is likely that the simpler expression has fewer local minima. There is also no need to check the chirality of the conformations produced. These advantages make the approach considerably faster.

After minimisation, the conformation is accepted if it passes the following criteria. The value of the cost function must be less than a user defined maximum (typically about 0.5 $Å^2$), and the substituent must not be clashing with the receptor, with the template or with itself. If the conformation fails these checks, the tweak routines are used to find an alternative conformation - the procedure is repeated until an acceptable geometry is located or a user definable number of attempts has been exceeded (see Murray (supra)).

The substituent still attached to the template is then optionally minimised using a molecular mechanics energy function. This is done in the presence of the receptor (which is treated as rigid) and a cut-off on the long range terms of 8Å is usually applied. An estimate of the strain energy in the receptor-bound conformation is obtained by performing the minimisation (starting from the tweak-generated geometry) in the absence of the receptor and subtracting from this energy, the intramolecular energy of the receptor-bound conformation. During these calculations, the template part of the molecule is held rigid. All molecular mechanics calculations may be done employing the fast and approximate 'Clean' forcefield developed by Hahn (J. Med Chem 38:2080 (1995)). Partial charges are calculated using the method of Gasteiger and Marsili (see Tetrahedron 36:3219 (1980)). The Clean forcefield bears many similarities to the 'generalised atom' forcefield incorporated in the Chem-X software (available from Chemical Design Ltd, Chipping Norton, UK) in that it does not rely on extended forcefield atom types. Only element type, hybridisation and bond type are used in calculating the energy of a system (see Hahn (supra)). A number of minor adjustments may be made in the implementation of the forcefield. The first is that all hydrogen atoms are treated specifically and are assigned an $sp^3$ atom type. The second is that van der Waals' radii for potential hydrogen-bond-forming atom pairs are scaled, typically by 0.8. It should be realised that the purpose of the Clean forcefield in the process of the invention is to provide a rough clean up of the substituents, which may possess distorted geometries caused by unrealistic torsion angles. The forcefield must be robust, in the sense that it must be able to cope with any chemistries that are given to it, and this is why a generalised atom forcefield is the most obvious choice. Additionally it must meet the approximate accuracy criteria, and in this context, the accuracy of the intermolecular terms is important. It was after analysis of intermolecular geometries obtained using Clean that the scaling of the hydrogen bonding van der Waals' radii was introduced. It is believed that the forcefield produces improved and reasonable geometries - at least when some portion of the molecule (here the template) is held fixed in the receptor.

The minimised conformation of the substituent (still attached to the template) is then assigned a score using a scoring function developed by Bohm for use in the de novo design program LUDI (see J. Comput.-Aided Mol. Des. 8: 243 (1994)). Böhm's scoring function permits an approximate calculation of the binding free energy of the substituent and template in terms of readily calculable quantities such as lipophilic contact surface area, the number and quality of hydrogen bonds formed and the number of rotatable bonds. Following Böhm, the form of the equaton used is

$$\Delta G_{binding} = \Delta G_0 + \Delta G_{hb} \Sigma_{hbonds} f\,(\Delta R,\,\Delta\alpha) + \Delta G_{ionic}\Sigma_{ionic} f(\Delta R,\,\Delta\alpha)$$

$$+\Delta G_{lipo}|A_{lipo}| + \Delta G_{rot}N_{rot}$$

where

$$f(\Delta R, \Delta\alpha) = f1(\Delta R)f2(\Delta\alpha)$$

and

$$f1(\Delta R) = \begin{cases} 1 & \Delta R \le 0.2\text{\AA} \\ 1 - (\Delta R - 0.2)/0.4 & \Delta R \le 0.6\text{\AA} \\ 0 & \Delta R > 0.6\text{\AA} \end{cases}$$

and

$$f2(\Delta\alpha) = \begin{cases} 1 & \Delta\alpha \le 30° \\ 1 - (\Delta\alpha - 30)/50 & \Delta\alpha \le 80° \\ 0 & \Delta\alpha > 80° \end{cases}$$

$f(\Delta R, \Delta\alpha)$ is a function which penalises hydrogen bonds whose geometry deviates from ideality. $\Delta R$ is the deviation of the H ... O/N hydrogen bond length from 1.9Å; $\Delta\alpha$ is the deviation of the hydrogen bond angle N/O-H... O/N from its ideal value of 180°. $\Delta G_0$ is a contribution to binding energy which is independent of interactions with the protein. Böhm suggests that this may be rationalised as a reduction in binding energy due to loss of translational and rotational entropy of the ligand. $\Delta G_{hb}$ describes the contribution from an ideal hydrogen bond and $\Delta G_{ionic}$ the contribution from an unperturbed ionic interaction. $\Delta G_{lipo}$ denotes the contribution from lipophilic interactions which is assumed to be proportional to the lipophilic contact surface between ligand and protein, $A_{lipo}$. Finally, $\Delta G_{rot}$ describes the loss of binding energy due to the freezing of internal degrees of freedom in the ligand. $N_{rot}$ is the number of acyclic $sp^3$-$sp^3$ and $sp^3$-$sp^2$ bonds excluding the rotations of terminal methyl and amine groups.

The values used for the various coefficients are those adopted by Bohm for the LUDI program: $\Delta G_0 = 5.4$, $\Delta G_{hb} = -4.7$, $\Delta G_{ionic} = -8.3$, $\Delta G_{lipo} = -0.17$ and $\Delta G_{rot} = 1.4$. The coefficients were obtained by fitting the equation to the activities for ligand-receptor binding where crystallographic structures for the complexes were available (although a few geometries were obtained by docking the ligands into the receptor). The accuracy of the function is not expected to be better than 1.5 orders of magnitude in the binding affinity.

Using this scoring function, it is possible to rank the accepted substituents according to the strength of interaction they are likely to make with the receptor by subtracting the pre-computed score for the template from the total score for the template-substituent combination.

Since the first-found conformation is not necessarily the highest scoring one available to the substituent, a user-specified number of acceptable conformations (typically 10 or more) will be sought and scored. After these conformations have been examined, the substituent geometry with the highest score is saved for future reference.

Once potential substituents have been located for each template attachment point, one can automatically enumerate the possibilities to produce the full library for all combinations of those substituents and the template. However, it is usually advisable to consider the substituent lists further so as to reduce the size of the enumerated library.

The output thus far for each template attachment point is a directory of substituent files each containing scoring and database information. A directory of substituents, the receptor structure and the template molecule are read in to a graphical visualisation package. This package may be designed to allow the user to scroll quickly through the substituent list in any order whilst displaying the file name and any molecular properties that are present in the substituent files (e.g the strain energy, the Böhm score or the components of the score). The properties may be displayed in a spreadsheet running alongside the molecular visualisation. Substituents can be visualised in isolation, with the template or with the receptor structure.

A set of substituent structures is treated as a list on which operations can be performed by the user. For instance one would probably want to store all structures with Böhm scores less than a given value in a new list of 'Good Scores'; one might also want to exclude all structures with high strain energies, and possibly remove bad structures judged by more subjective criteria (e.g. bad chemistries or geometries). The user can have full control over which list of structures are displayed. At any time the user can write a list to a new or old directory or remove a list from an old directory.

Coupled to the list functionality is a clustering facility which allows one to cluster a specified list on the basis of 2D chemical functionality. The clustering may be based on similar functionality available in PRO_LIGAND which measures similarity by Tanimoto coefficients derived from bit string representations of the chemical structures (see Willett, J. Chem. Inf. Comput. Sci. 26:109 (1986) and Barnard, J. Chem. Inf. Comput. Sci. 32:644 (1992)). The bit strings may be specified by 172 atom-centred fragments generated from an analysis of 5000 structures in the Cambridge Structural Database (see Allen, J. Chem. Inf. Comput. Sci. 31:187 (1991)). Several different clustering algorithms are available, and one may use a hierarchical clustering method such as Complete Linkage or Ward's. (The number of structures clustered may typically be about 100 or less, so CPU time is not an issue.) A number of tools are available to help decide on the appropriate number of clusters for the specified lists. The output from the clustering is a new set of lists each containing an individual cluster. These can be browsed and operated on as described above. Whilst the clustering is not always perfectly in line with chemical intuition, it is an extremely useful way of navigating through and keeping track of a fairly large number of substituents.

The final facility provided by the molecular browser is to rescore a list of substituents using the empirical Böhm score. Rescoring in this way is practical because tens of structures can be scored per second and is useful because information gained during the scoring can be used to provide a graphical representation of the score. Hydrogen bonds or ionic interactions are located, marked and annotated with the contribution they make to the predicted binding affinity. This saves a lot of time in deciding which hydrogen bonds are formed and how good they are. It also points out hydrogen bonds which may be contributing to the score in an unrealistic way. Bonds that are considered rotatable are also marked so that the user can see which bonds are (or are not) contributing to the score. Finally, the grid used to establish the lipophilic contribution to the score is displayed graphically. Relevant grid points fall into several categories:

- lipophilic ligand atom in contact with lipophilic receptor atom

- lipophilic ligand atom in contact with polar receptor atom (or vice versa)

- polar ligand atom in contact with polar receptor atom - lipophilic ligand atom in contact with nothing (i.e. solvent)

- polar ligand atom in contact with nothing (i.e. solvent)

- volume of ligand

The user can colour each of these grid point types, though in practice, we have tended to use colours for the first three types only. The visualisation is useful because it displays aspects of ligand-receptor contact which are often difficult to assess quickly from looking at the complex alone.

After application of these tools a smaller set of substituents is decided on for each of the template attachment points. The aspects which are considered in producing this list are:

**2D diversity** Using the clustering tools and chemical knowledge, a diverse set of substituents may be chosen. For example, if there are 10 fluorinated derivatives of phenylalanine only one need be chosen. Exploration of different chemistries is important because the scoring functions can only be expected to deliver approximate accuracy in the prediction of the binding affinity.

**3D contacts** It is important to look at the contacts a substituent is predicted to make with the receptor and to form a judgement as to whether these seem reasonable or not. In particular, substituents which have a large amount of polar-nonpolar contact are suspect. There should also be an awareness of 3D diversity and there should be an attempt to target molecules which explore different forms of receptor contact to make up for deficiencies in the scoring criteria.

**synthetic considerations** There should be a consideration of synthetic feasibility. Although the strategy of making single compounds by the most appropriate protocol means that a larger diversity of substituents are synthetically accessible, there will still be some substituents which contain functionalities that are difficult to incorporate in any synthetic protocol. Additionally, where one compound is to be chosen from several similar possibilities, choices could be made on the basis of ease of availability or price of the compounds.

**scores** The scores of the substituents (e.g. Böhm scores, forcefield energies, etc.) can be used to choose preferred substituents from among lists of similar compounds.

The process of combinatorial enumeration simply involves forming a list of all the remaining substituents at each R-group position and then creating all possible combinations of them. Thus, given a template with three R-group positions and three substituents for each, the combinatorial enumeration procedure will produce 27 different molecules. The geometries produced are based on the highest scoring geometries of the corresponding substituents. The resulting molecules are stored for further analysis or transfer to a 3D database.

The resulting molecules can be, but are not usually, minimised with the Clean forcefield and are then rescored in the same manner as the substituents. Estimated logP and molecular weight are also routinely calculated for the complete molecules.

In our applications, the complete molecules have also been subjected to evaluation using the CFF95 forcefield in Discover (available from Molecular Simulations Inc, San Diego, California, US). Simplified cut down models of the receptor are used and minimisation and molecular dynamics are used to assess the quality of the designs. If the designs are reasonably stable during dynamics and possess high scoring snapshots then they are considered suitable for synthesis.

The final decision about which molecules to synthesise is made by considering all the data collected for the substituents and enumerated molecules. The full library could be synthesised, or selected molecules can be chosen from the full library. The possibility of experimental design to choose the best candidates has been explored. The method initially used was D-optimal design which attempts to maximise the coverage of a specified property space in a subset of molecules chosen from a larger library. In our explorations, the spread in the following properties was approximately maximised:

- the substituents from which each library member was derived

- estimated value of logP for each library member

- the hydrogen bond, the rotatable bond and lipophilic contributions to the Böhm score for each library member

Several constraints can be imposed on the design such as inclusion or exclusion of compounds which are outside a specified range of a molecular property. The general conclusion of this application of experimental design was that although it was useful, practical considerations, such as ease of synthesis of particular classes of compounds from the full library, were usually more important.

Most of the computationally intensive routines of the operating software for the process of the invention may be written in Fortran, the data structure and data handling code in C, and the drivers and user interface parts in Global. Global is a proprietary interpreted language designed for application to computer-aided molecular design. The main use of Global is that, together with the chemical utilities and their associated data structure routines, it provides a flexible environment for the operation of the process of the invention. A language which allows high order chemical design features and user input to be expressed succinctly and naturally makes the methods easy to program, amend and debug. Global also makes mundane tasks such as IO and memory management straightforward, and frees the programmer to concentrate on the chemical design aspects of a programming task. Because there is no compilation for the interpreted language it is easy to adapt the drivers and run them interactively or in batch mode. The user can either treat the GLOBAL files as input decks in the traditional sense or, if they have more confidence, can make fairly significant changes to the order of operation of the drivers, introducing different screens for the substituents as they see fit. Higher level languages have shown their worth before in CAMD applications as illustrated by TRIPOS's SPL language or the various languages offered to MSI users.

In the method of the invention, the drug compound may if desired be formulated for administration, eg. via parenteral or enteral routes, for example orally, rectally, nasally, transdermally, by injection or infusion, or into the lungs. Typical administration forms include tablets, powders, capsules, suppositories, syrups, sprays, solutions, dispersions, suspensions, emulsions and gels. Such compositions may contain conventional pharmaceutically acceptable carriers and excipients, eg. water for injections, physiological saline, buffers, sweeteners, dispersants, bulking agents, etc.

## EXAMPLE

The generation of a Library of thrombin inhibitors is described as an example of the present invention.

Thrombin is a trypsin-like serine protease recognised as a key enzyme within the coagulation cascade. Its primary action is catalysis of the conversion of soluble fibrinogen to insoluble fibrin, which is the basis of thrombus formation and blood clotting. In addition, thrombin has several other roles in the control of pro- and anti-coagulant pathways in the coagulation cascade, inducing platelet aggregation, and more general signalling roles via activation of a thrombin receptor. As thrombin is the final step in both the extrinsic and intrinsic clotting cascades, it has attracted much attention as a therapeutic target. Modulation of thrombin activity may be of use to prevent inappropriate thrombus formation, for example as a general anticoagulant as an adjunct to surgery or as a prophylaxis in various cardiovascular disorders such as myocardial infarction and unstable angina. Direct competitive inhibition of thrombin has been pursued by several pharmaceutical companies in an effort to obtain a new class of anticoagulants and antithrombotics, potentially with good oral bioavailability and improved efficacy and toxicity when compared with existing drugs.

The present example relates to the design of a library of novel thrombin inhibitors which are potential drug candidates. At this stage the quality of the designs was assessed in terms of an in vitro assay of thrombin inhibition. Successful designs may be selected on the basis of the measured inhibition constant ($K_i$). In addition, the selectivity of the compounds towards thrombin may be assessed by performing enzyme inhibition assays versus structurally-related serine proteases, such as trypsin and Factor Xa. In general, enzyme specificity is an important consideration because an intended thrombin inhibitor may also inhibit fibrinolytic enzymes and hence exert an undesired thrombotic effect.

The first stage in the application of the process was the identification of an appropriate template structure and an associated synthetic strategy. This was achieved by analysis of known thrombin inhibitors in order to identify chemical moieties which appear to contribute favorably to binding. The source of the data was the Brookhaven protein database. From the available thrombin-ligand complexes it was decided to select as a template the proline moiety from the inhibitor PPACK (D-phenylalanyl-prolyl-arginyl-chloromethylketone).

This was chosen for several reasons. Previous analysis of SAR data for thrombin had highlighted PPACK as an effective inhibitor bound at the active site (that is the site at which the catalytic hydrolysis of the peptide substrate occurs). The activity of PPACK was believed to be the result of making favorable interactions with several distinct regions of the active site, most importantly two hydrophobic pockets (labelled as distal and proximal to the catalytic amino-acid residues) and a polar pocket (the arginine-binding pocket or in enzyme terminology the S1 pocket). Proline was considered a good choice for a template because it fulfilled the design criteria that it should make some favorable interactions in itself, and also allow the positioning of substituents which will also make favorable interactions. Analysis of the X-ray structure revealed that proline makes good interactions with the proximal hydrophobic pocket and allows the positioning of a potential library of substituents which are likely to make good interactions with the remaining two pockets. In the absence of an X-ray structure these assumptions would have to be made on the basis of modelling the enzyme-template complex.

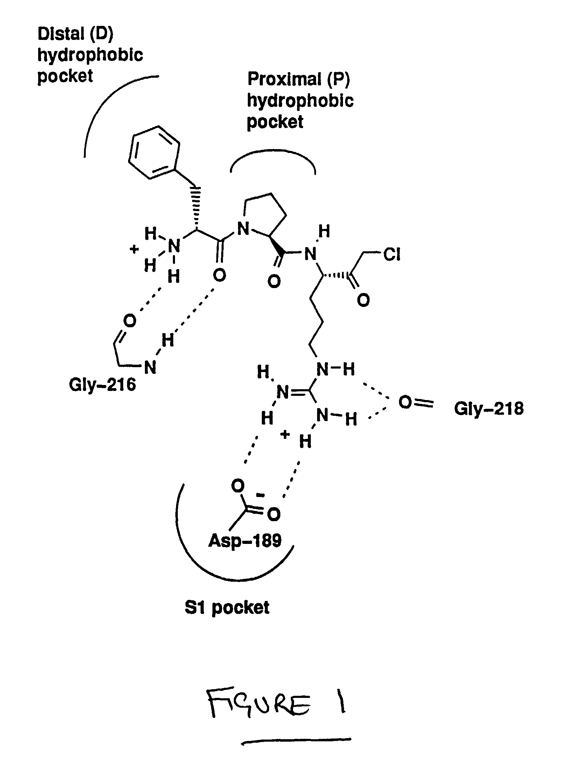

The structure of PPACK and some of its key interactions with thrombin are shown in Figure 1. It was the intention to design a library of reversible inhibitors exploring a diverse set of substituents in the D-Phe and Arg positions.

Several sets of substituent lists were prepared using different design criteria. Initially, the N-terminus on proline was targeted with starting reagents that possessed a carboxylic acid (to form a peptide bond with the template), and a hydrogen bond donor plus a hydrophobic group (to form contacts with the D pocket). This was later augmented by a list of sulphonic acids and sulphonyl chlorides (to form a sulphonamide bond with the template). The C-terminus was initially targeted with starting reagents that possessed bis-amines (to form a peptide bond with the template and hydrogen bonds in the S1 pocket). This was augmented by a list of amines with aromatic nitro compounds used as 'protected' anilines; and a list in which amines were 'protected' as nitrile compounds. In all cases, there were 2D and 3D constraints imposed when searching through the ACD. Two positionings of the template in an associated receptor conformation were used. The first was derived directly from the proline position in the crystal structure of the covalently bound PPACK, and the second was derived from a computational simulation of a non-covalently bound analogue of PPACK.

Table 1 gives some details of the numbers of compounds considered at each stage of the process for the second template position (the results from the first template position are very similar). For the sake of clarity, the results of only one substituent list at each template attachment point are given. The 2D search was not fully refined and includes many reagents which are not practicable with any simple synthetic route. It also includes many substituents which would be ruled out of a single protocol combinatorial approach yet have been successfully included in our final compound set. It is clear that even after a thorough application of 3D database searching, the virtual library size is still enormous and receptor screening and scoring are required to reduce it to a manageable number.

Table 1:

| statistics for number of molecules considered at each stage. The list of substituents for the Arg position were primary/secondary amines (plus hydrazines) for the 2D search, and bis-amines separated by 5-8Å for the 3D search. For the Phe position the list was carboxylic acids for the 2D search, and hydrophobic carboxylic acids with a donor group 2-3Å away for the 3D search. | | |
|---|---|---|
| Stages | No. of accepted substituents | | No. of compounds in Virtual Combinatorial |
| | **Arg** position | **Phe** position | Library |
| After 2D ACD screen | 4262 | 8803 | 37518386 |
| After 3D ACD screen | 894 | 437 | 390678 |
| After receptor screen | 144 | 145 | 20880 |
| After binding affinity screen | 65 | 81 | 5265 |
| After strain energy screen | 53 | 71 | 3763 |
| Selected synthesis candidates | 9 | 8 | 72 |

The resulting substituent lists were then more thoroughly evaluated using: 2D chemical diversity; visualisation of the 3D contacts made by the substituents (those which interacted with different parts of the receptor were especially targeted); further computational evaluation of the predicted binding affinities, interaction energies and physical properties of the substituents and their enumerated counterparts; and further consideration of synthetic feasibility.

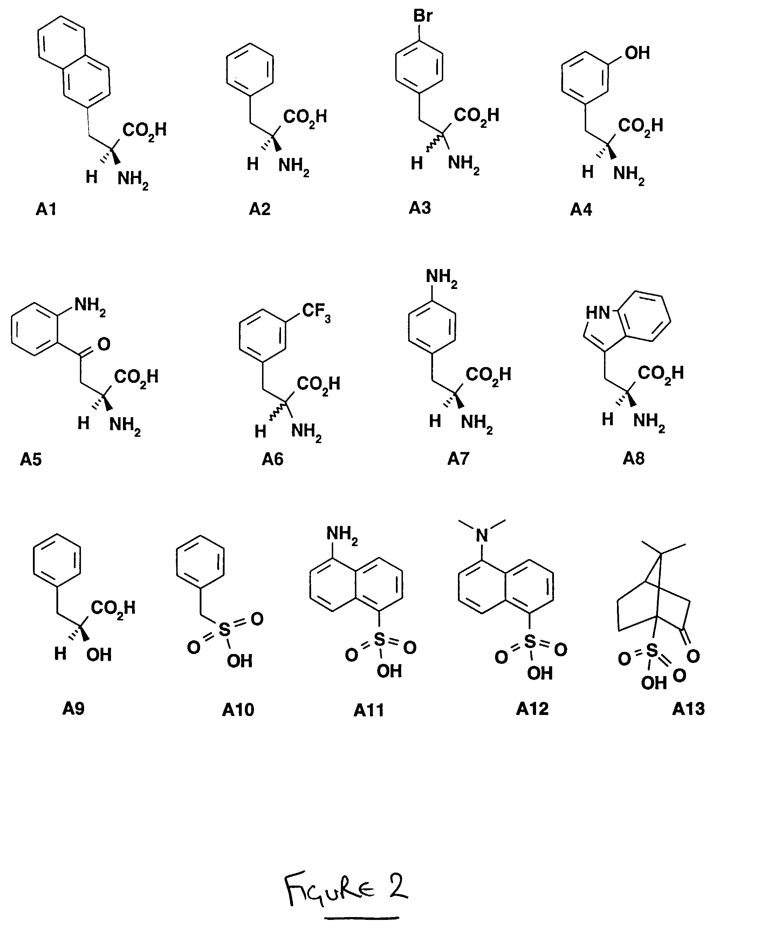

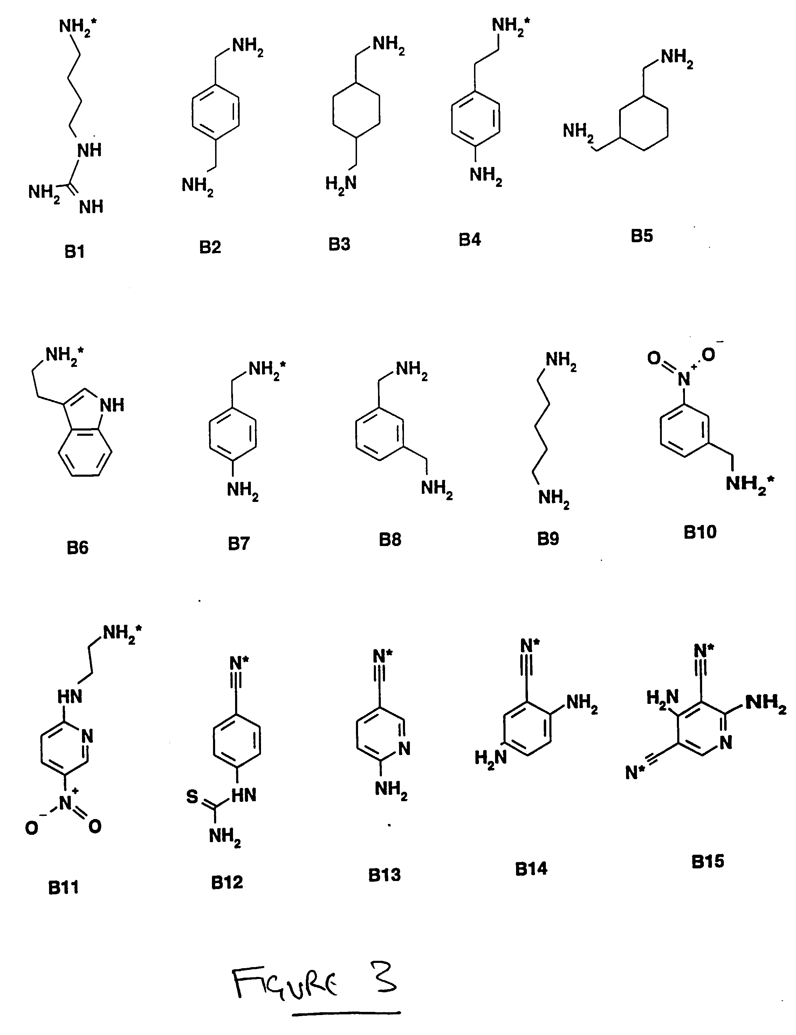

Figures 2 and 3 give examples of the starting materials used in the synthesis of library members based around the proline template. Not all possible members of the library were enumerated. All substituents at the Phe position were enumerated with the prolinyl-agmatine moiety (i.e. representing a good Arg position substituent) and all substituents at the Arg position were coupled to the D-phenylalaninyl-proline moiety (i.e. representing a good Phe position substituent). In addition a full array involving reagents A1-A4 and B1-B4 was synthesised. The basic synthetic protocols were modified as necessary to take account of the wide diversity of functionality in the starting materials. In particular, a solid phase approach was used with the bis-amines (B2, B3, B5, B8 and B9) and solution methods were used for the others. The nitrile compounds were reduced in advance of coupling. All solution phase routes proceeded via coupling of the activated acids to the proline benzyl ester which allows for deprotection via hydrolysis or hydrogenation dependent upon substituents within the acid.

The D-amino acid analogues of array A had free amino groups protected with Boc. Where B was a symmetrical bis-amine, the amine was attached to acid-labile chlorotrityl resin and the resin washed with dichloromethane and DMF. Fmoc proline was attached using TBTU/DIPEA activation (2 eq). After deprotection of the amino group with 20% piperidine in DMF, the Boc protected A component was coupled as before. The product was cleaved from the resin with 10% TES in TFA (30 min), evaporated to dryness and triturated with diethyl ester to give the crude product. Where B was an asymmetric amine, the Boc protected A component was coupled to proline benzyl ester (1 eq) by activation with TBTU/TEA. Hydrolysis of the benzyl ester using NaOH (1.1 eq) in acetone/water (1:1) yielded dipeptide acid which was pre-activated (TBTU/TEA) and reacted with the amine (1.1 eq) in DMF (or DMF/water (1:1) for water soluble B components). Extraction of the product followed by deprotection (5% aq TFA), evaporation and trituration with diethyl ether, yielded the crude product.

The other members of the A array (sulphonic acids, sulphonyl chlorides and $\alpha$-hydroxy acid) were attached without further protection. Sulphonyl chlorides were reacted with proline benzyl ester in the presence of TEA (2 eq). The ester product was hydrolysed as above and B1 coupled via TBTU/TEA pre-activation as above. The product was extracted with methanol after evaporation of the reaction mixture to dryness.

The 'protected' B compounds were coupled as described above for asymmetric amine B compounds after appropriate reduction: $H_2$, Pd/C in the case of nitro groups, catalytic transfer hydrogenation (hydrazine hydrate, ethanol, Pd/C, 85°C) for B12, and LAH for the remaining nitrile compounds.

The compounds were tested for inhibition of thrombin and trypsin using a colorimetric microplate assay with synthetic peptide substrates as described by Tapparelli, J. Biol. Chem. 268:4734 (1993). In general the compounds were tested as crude products and the more active compounds were purified, and accurate $K_i$ were experimentally determined. The results are given in Table 2, and show that almost all of the compounds were active against the two enzymes with several compounds showing selectivity for thrombin. The most active compound (A3B1) has a $K_i$ of 41 nanomolar.

Table 2:

| Inhibition results for molecules synthesised. The $K_i$ values are in micromolar. Where values are in parentheses, only the crude compounds were tested. Generally, activity increased by between 3 and 10 times when pure samples were used. Where no value is given, the molecules were not active. The errors in the calculation of the $K_i$ for purified compounds are less than 10%. | | | | | |
|---|---|---|---|---|---|
| Compound | Thrombin | Trypsin | Compound | Thrombin | Trypsin |
| A1B1 | 0.56 | 0.95 | A1B2 | (20) | (0.6) |
| A1B3 | (30) | (10) | A1B4 | (100) | (-) |
| A2B1 | 0.12 | 0.25 | A2B2 | 0.83 | 0.19 |
| A2B3 | 2.8 | 8.8 | A2B4 | (50) | (-) |
| A3B1 | 0.041 | 0.60 | A3B2 | 0.30 | 0.95 |
| A3B3 | 1.3 | 58 | A3B4 | (30) | (-) |
| A4B1 | 1.4 | 1.9 | A4B2 | (22) | (0.6) |
| A4B3 | (50) | (13) | A4B4 | (200) | (-) |
| A2B5 | (10) | (40) | A2B6 | (90) | (-) |
| A2B7 | 0.71 | 58 | A2B8 | (20) | (6) |
| A2B9 | 0.69 | 1.5 | A2B10 | 4.0 | 590 |
| A2B11 | - | - | A2B12 | - | - |
| A2B13 | - | - | A2B14 | 48 | - |
| A2B15 | - | - | | | |
| A5B1 | 2.9 | 0.12 | A7B1 | 0.28 | 1.0 |
| A851 | 1.6 | 0.67 | A9B1 | 0.53 | 0.52 |
| A10B1 | (-) | (9) | A12B1 | (-) | (1) |
| A13B1 | (200) | (90) | | | |

At the Phe position, the best scoring substituents were aromatic D-amino acids, which reflects the strict 3D constraints imposed by the thrombin active site and the need to form good hydrophobic contacts if high affinity is to be achieved. The process of the invention did produce non-amino acid solutions but these scored poorly. The best substituent was the p-Br-D-Phe (A3) which is three times more active than the simple Phe derivative. The available starting material was a racemic mixture and the resulting diastereoisomers were separated by HPLC. As predicted, one of the diastereoisomers was at least 100 times less active than the other. Of particular interest are the substituents with polar functionality (A4, A5 and A7) which have not been thoroughly explored before in PPACK analogues. These were selected because they were predicted to form additional hydrogen bonds, which if not contributing to affinity, could enhance selectivity. The poor activity of the sulphonamide derivatives against thrombin was not particularly surprising since the design criteria for this substituent list omitted a hydrogen bond to Gly-216. Despite this drawback, the syntheses were justified because the sulphonamides increased the chemical diversity and allowed the exploration of different modes for the hydrophobic pocket.

At the Arg position, the most active base is agmatine, as expected, since the guanidino group can make excellent contacts with Asp-189 and Gly-218 at the bottom of the S1 subsite. However there is great incentive to diverge from the arginine-like chemistry because of its pharmacokinetic properties and side-effect profile. Di-amino pentane (B9) is active, as would be expected for a lysine analogue (see Brady, Bio Med. Chem 8:1063 (1995)). The other bis-amines also have respectable activity, which is of interest because good hydrophobic contacts in this pocket may increase affinity and selectivity (see Deadman, J. Med Chem 38:1511 (1995)). The activity of the short aniline (B7) is particularly interesting. It is unlikely that this substituent is long enough to interact directly with Asp-189 (although there could be a mediating water molecule), instead it is predicted to form hydrogen bonds to Gly-219 and Ala-190. It was the activity of this compound which caused us to explore different anilines using the functional group transformation strategy.

Viewed from a second aspect the invention also provides novel active compounds identified by the process of this invention. Thus the compounds for which non-parenthesized activity values are given in Table 2 above are deemed to fall within the scope of the invention as are all other active PPACK analogs incorporating the 'successful' substituents that characterise reagents A3, A4, A5, A7 and B7.

**Claims**

1. A process for drug candidate identification, said process comprising the steps of:

(1) obtaining a computerised representation of the three-dimensional structure of a binding site on the surface of a biological macromolecule;

(2) generating a computerised model of the functional structure of said binding site which may be used to identify favourable and unfavourable interactions between the binding site and a drug candidate molecule;

(3) identifying a molecular fragment capable of placement within said binding site and capable of carrying at least one substituent group, said molecular fragment either being capable of being synthesized from reagent compounds accessible in substituted form whereby to import said substituent groups on synthesis of said molecular fragment or being present in an accessible reagent compound capable of substitution with said substituent groups by reaction with further accessible reagent compounds;

(4) generating a set of lists of accessible reagent compounds, the lists being such that a combination of compounds taken from each list may be reacted to produce a candidate compound comprising said molecular fragment carrying a plurality of substituent groups thereby generating a first virtual library of candidate compounds being the theoretical set of compounds producible by reaction of the members of said lists, each member of each list comprising a component common to the other members of that list and a component unique within that list;

(5) for each said list limiting the number of members thereof using a first set of exclusion rules thereby to generate a restricted second virtual library of candidate compounds, the operation of said first set of rules involving for each member of each list computerised comparison for favourable or unfavourable interactions between said computerised model and a structure comprising said molecular fragment and a substituent deriving from the unique component within said list of that member, the molecular fragment and the computerised model being held in fixed spatial relationship to each other for said comparison;

(6) evaluating and ranking by computer the members of said second virtual library for favourable and unfavourable interactions with said computerised model and thereby generating a restricted third virtual library of candidate compounds ranked as having favourable interactions;

(7) optionally, selecting from said third virtual library at least one further molecular fragment and repeating steps (4), (5) and (6) to generate an alternative third virtual library;

(8) screening said third virtual library using a second set of exclusion rules thereby to generate a restricted fourth virtual library of candidate compounds comprising compounds which are candidates for synthesis and experimental evaluation for drug efficacy;

(9) synthesizing some or all candidate compounds of said fourth virtual library to produce a candidate compound library;

(10) experimentally evaluating the compounds of said candidate compound library for drug efficacy;

(11) analysing the experimental efficacy data generated in step (10) for structure-activity relationship information;

(12) using the information derived in step (11) selecting a revised set of lists of accessible reagent compounds, said lists being expanded to include selected reagents not present in the restricted lists generated in step (5) and optionally restricted to exclude selected reagents present in the restricted lists generated in step (5);

(13) repeating steps (6) and (7) to identify further compounds which are candidates for synthesis and experimental evaluation for drug efficacy;

(14) synthesising and experimentally evaluating said further compounds for drug efficacy;

(15) if required repeating steps (11) to (14) one or more times;

(16) identifying as a lead candidate a compound synthesized and experimentally evaluated as above.

2. A process according to claim 1 wherein in step (7) at least one further molecular fragment is selected from the third virtual library, whereafter steps (4), (5) and (6) are repeated to generate an alternative third virtual library which is subsequently screened in step (8).

3. A process according to either of claims 1 and 2 wherein in step (12) said revised set of lists of accessible reagents is selected to include reagents excluded from the restricted lists generated in step (5) for being analogs of reagents included in said restricted lists.

4. A process according to any one of claims 1 to 3 wherein in step (12) said revised set of lists of accessible reagents is selected to include reagents excluded from the restricted lists generated in step (5) for involving complex transformation in their synthesis from commercially available reagents.

5. A process according to any one of claims 1 to 4 wherein in step (12) said revised set of lists of accessible reagents is selected to include reagents excluded from the restricted lists generated in step (5) for being produced in low yield in their synthesis from commercially available reagents.

6. A process according to any one of claims 1 to 5 wherein in step (12) said revised set of lists of accessible reagents is selected to include reagents excluded from the restricted lists generated in step (5) for requiring significant purification following their synthesis from commercially available reagents.

7. A process according to any one of claims 1 to 6 wherein in step (12) said revised set of lists of accessible reagents is selected to include reagents excluded from the restricted lists generated in step (5) for being expensive.

8. A process according to any one of claims 1 to 7 wherein in step (1) said representation is derived from X-ray crystallographic data for said macromolecule.

9. A process according to any one of claims 1 to 8 wherein in step (4) said lists of accessible reagents are generated from a computer database of available chemicals.

10. A process according to claim 9 wherein in step (4) said lists of accessible reagents are supplemented to include reagents accessible by transformation of reagents identified from said database.

11. A process according to any one of claims 1 to 10 wherein the model generated in step (2) comprises a representation of those regions of the binding site capable of interaction with a molecule placed in said binding site the said regions being identified according to the nature and geometry of said interaction.

12. A process according to any one of claims 1 to 11 wherein in step (5) said computerised comparison for a reagent involves in sequence: (i) carrying out a subgraph isomorphism check to establish a match between said unique component of said reagent and said computerised model, (ii) rejecting reagents for which no match can be found, (iii) verifying the match for non-rejected reagents by torsional optimization of the rotatable bonds in the unique component, (iv) calculating the compatibility between the computerised model and a structure comprising the molecular fragment and the substituent deriving from the unique component of the reagent in the confirmation predicted by step (iii), (v) optionally repeating steps (iii) and (iv) to seek a conformation with enhanced compatibility, (vi) rejecting reagents for which a preselected degree of compatibility is not found in steps (iv) and (v), (vii) determining a score indicative of a minimum energy level for said structure within said computerised model with the structure and position of said molecular fragment held constant, and (viii) ranking the reagents in a list according to the scores determined in step (vii).

13. A process according to claim 12 wherein in step (vii) scores indicative of strain energy and contributions to energy level of individual interactions of components of said structure with said computerised model are also determined and reagents are rejected if such scores exceed pre-selected limits indicative of undesirable conformation or interaction.

14. Novel active compounds identified by a process according to any one of claims 1 to 13.

19

**15.** A method of manufacturing a drug substance, said method comprising the steps of:

(1) obtaining a computerised representation of the three-dimensional structure of a binding site on the surface of a biological macromolecule;

(2) generating a computerised model of the functional structure of said binding site which may be used to identify favourable and unfavourable interactions between the binding site and a drug candidate molecule;

(3) identifying a molecular fragment capable of placement within said binding site and capable of carrying at least one substituent group, said molecular fragment either being capable of being synthesized from reagent compounds accessible in substituted form whereby to import said substituent groups on synthesis of said molecular fragment or being present in an accessible reagent compound capable of substitution with said substituent groups by reaction with further accessible reagent compounds;

(4) generating a set of lists of accessible reagent compounds, the lists being such that a combination of compounds taken from each list may be reacted to produce a candidate compound comprising said molecular fragment carrying a plurality of substituent groups thereby generating a first virtual library of candidate compounds being the theoretical set of compounds producible by reaction of the members of said lists, each member of each list comprising a component common to the other members of that list and a component unique within that list;

(5) for each said list limiting the number of members thereof using a first set of exclusion rules thereby to generate a restricted second virtual library of candidate compounds, the operation of said first set of rules involving for each member of each list computerised comparison for favourable or unfavourable interactions between said computerised model and a structure comprising said molecular fragment and a substituent deriving from the unique component within said list of that member, the molecular fragment and the computerised model being held in fixed spatial relationship to each other for said comparison;

(6) evaluating and ranking by computer the members of said second virtual library for favourable and unfavourable interactions with said computerised model and thereby generating a restricted third virtual library of candidate compounds ranked as having favourable interactions;

(7) optionally, selecting from said third virtual library at least one further molecular fragment and repeating steps (4), (5) and (6) to generate an alternative third virtual library;

(8) screening said third virtual library using a second set of exclusion rules thereby to generate a restricted fourth virtual library of candidate compounds comprising compounds which are candidates for synthesis and experimental evaluation for drug efficacy;

(9) synthesizing some or all candidate compounds of said fourth virtual library to produce a candidate compound library;

(10) experimentally evaluating the compounds of said candidate compound library for drug efficacy;

(11) analysing the experimental efficacy data generated in step (10) for structure-activity relationship information;

(12) using the information derived in step (11) selecting a revised set of lists of accessible reagent compounds, said lists being expanded to include selected reagents not present in the restricted lists generated in step (5) and optionally restricted to exclude selected reagents present in the restricted lists generated in step (5);

(13) repeating steps (6) and (7) to identify further compounds which are candidates for synthesis and experimental evaluation for drug efficacy;

(14) synthesising and experimentally evaluating said further compounds for drug efficacy;

(15) if required repeating steps (11) to (14) one or more times;

(16) identifying as a lead candidate a compound synthesized and experimentally evaluated as above;

(17) manufacturing the compound identified in step (16) above; and, optionally,

(18) admixing the compound manufactured in step (17) above with at least one pharmaceutically acceptable carrier or excipient.

FIGURE 1

A1  A2  A3  A4

A5  A6  A7  A8

A9  A10  A11  A12  A13

FiguRE 2

FIGURE 3